# EUROPEAN PATENT SPECIFICATION

(11) **EP 3 648 745 B1**
(45) Date of publication and mention of the grant of the patent: **26.06.2024**
(21) Application number: 18860031.6
(22) Date of filing: 28.09.2018
(51) Int. Cl.: A61K 9/20, A61K 9/28, A61K 9/48, A61K 31/4439

(54) **PHARMACEUTICAL COMPOSITION INCLUDING MULTI-UNIT SPHEROIDAL TABLET CONTAINING ESOMEPRAZOLE AND SPHEROIDAL PHARMACEUTICALLY ACCEPTABLE SALT THEREOF, AND METHOD OF PREPARING THE PHARMACEUTICAL COMPOSITION**
PHARMAZEUTISCHE ZUSAMMENSETZUNG MIT KUGELFÖRMIGER TABLETTE AUS MEHREREN EINHEITEN MIT ESOMEPRAZOL UND KUGELFÖRMIGES, PHARMAZEUTISCH VERTRÄGLICHES SALZ DAVON UND VERFAHREN ZUR HERSTELLUNG DER PHARMAZEUTISCHEN ZUSAMMENSETZUNG
COMPOSITION PHARMACEUTIQUE COMPRENANT UN COMPRIMÉ SPHÉROÏDAL À UNITÉS MULTIPLES CONTENANT DE L'ÉSOMÉPRAZOLE ET UN SEL DE QUALITÉ PHARMACEUTIQUE DE CELUI-CI, ET PROCÉDÉ DE PRÉPARATION DE LA COMPOSITION PHARMACEUTIQUE

(30) Priority: 28.09.2017 KR 20170126426
(43) Date of publication of application: 13.05.2020
(73) Proprietor: Hanmi Pharm. Co., Ltd., Hwaseong-si, Gyeonggi-do 18536 (KR)
(72) Inventor: CHO, Hyuk Jun, Suwon-si Gyeonggi-do 16358 (KR); KIM, Min Wook, Suwon-si Gyeonggi-do 16359 (KR); IM, Ho Taek, Yongin-si Gyeonggi-do 16909 (KR); KIM, Yong Il, Suwon-si Gyeonggi-do 16325 (KR)
(74) Representative: Marks & Clerk LLP
(86) International application number: PCT/KR2018/011528
(87) International publication number: WO 2019/066555

(56) References cited:
- WO-A1-2018/021772
- WO-A1-2018/080104
- WO-A2-2005/009410
- WO-A2-2005/051362
- KR-A- 20100 078 462
- KR-A- 20140 076 998
- KR-A- 20180 011 624
- US-A1- 2015 098 992
- US-A1- 2015 098 992
- LOPES, C. M. et al.: "Compressed mini-tablets as a biphasic delivery system", International Journal of Pharmaceutics, vol. 323, 2006, pages 93-100, XP025113222, DOI: doi:10.1016/j.ijpharm.2006.05.063

## Description

### Technical Field

One or more embodiments relate to a pharmaceutical composition including a formulation in a form of a multi-unit spheroidal tablet (MUST) containing esomeprazole and pharmaceutically acceptable salt thereof and a method of preparing the pharmaceutical composition.

### Background Art

Esomeprazole ((S)-5-methoxy-2-[(4-methoxy-3,5-dimethylpyridine-2-yl)methysulfinyl]-3H-benzimid azole) is a compound represented by Formula 2, which is an (S)-optical isomer of omeprazole represented by Formula 1: 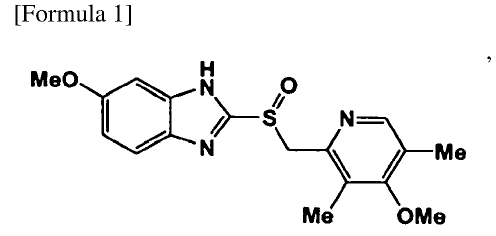 and

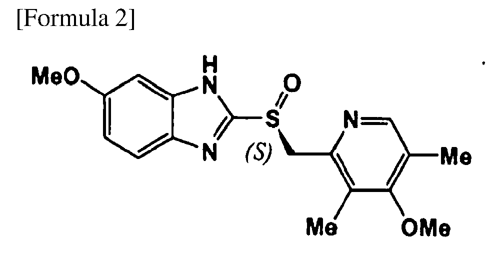

Proton pump inhibitors, such as esomeprazole, have an effect of inhibiting gastric acid secretion in mammals, including humans, by regulating gastric acid secretion at the final stage of the acid secretion pathway. Thus, proton pump inhibitors may be used for prevention and treatment of diseases related to excessive secretion of gastric acid such as gastroesophageal reflux disease such as reflux esophagitis, gastritis, duodenitis, gastric ulcer, duodenal ulcer, and peptic ulcer.

On the other hand, because esomeprazole is susceptible to dissociation or modification under acidic conditions, oral formulations containing esomeprazole need to be delivered to the gastrointestinal tract where pH is almost neutral, and rapid absorption thereof may occur. To this end, a formulation, in which exposure to gastric acid in stomach is prevented, and an enteric coating layer is formed for absorption in intestines, has been developed. After a lapse of 12 hours from taking conventional esomeprazole, gastric acid was secreted, and the pH was lowered, thus causing symptoms such as heartburn.

In addition, a core of conventional esomeprazole was limited to pellet formulations. Such pellet formulations have poor production reproducibility, low yield, and problems with esomeprazole stability and residual solvents due to drug coating.

Therefore, it is necessary to develop a new formulation of esomeprazole which has a double release pattern to improve drug compliance of a patient and drug efficacy and may improve production reproducibility and yield,

US2015/098992 A1 discloses a hard capsule composite formulation comprising 4 to 40 MUSTs, each with a diameter of 1 to 4 mm and having two or more pharmaceutically active ingredients, such as esomeprazole. The composite formulation may further comprise pharmaceutically acceptable additives such as a disintegrating agent. Said disintegrating agent is possibly selected from the group consisting of crospovidone, sodium starch glycolate, croscarmellose sodium, low-substituted hydroxypropyl cellulose, starch, alginate, or its sodium salt or a mixture thereof.

### Disclosure of Invention

### Technical Problem

Provided is a pharmaceutical composition which is rapid in dissolution of esomeprazole or a pharmaceutically acceptable salt thereof.

Provided is a method of preparing a pharmaceutical composition which is rapid in dissolution of esomeprazole or a pharmaceutically acceptable salt thereof.

### Solution to Problem

The invention is defined by the scope of the claims.

It provides a pharmaceutical composition comprising a core in a form of multi-unit spheroidal tablet (MUST) containing esomeprazole or a pharmaceutically acceptable salt thereof and a disintegrant, wherein the disintegrant is low-substituted hydroxypropyl cellulose, the content of the low-substituted hydroxypropyl cellulose being in a range of about 12 percent by weight (wt%) to about 30 wt%, based on a total weight of the core, wherein the pharmaceutical composition is dissolved by 50 percent (%) or more within 15 minutes of a dissolution test in presence of simulated intestinal fluid at 25 revolutions per minute (rpm), when a dissolution test takes place using the paddle method.

The esomeprazole or a pharmaceutically acceptable salt thereof may be used without any limitation as long as it is commonly used in the art. Examples thereof include a metal salt, e.g., a magnesium (Mg) salt, a strontium (Sr) salt, a lithium (Li) salt, a sodium (Na) salt, a potassium (K) salt, or a calcium (Ca) salt, of esomeprazole, or an ammonium salt of esomeprazole. For example, a pharmaceutically acceptable salt of the esomeprazole may be a Mg salt or Sr salt of esomeprazole.

The esomeprazole or pharmaceutically acceptable salt thereof may be an anhydride or a hydrate.

The esomeprazole or pharmaceutically acceptable salt thereof may be in a form of a dry granule. An angle of repose of the dry granule may be about 40° or less.

A MUST may be a mini-tablet of a spherical shape or a nearly cylindrical shape. The mini-tablet may be a small tablet. A diameter of the mini-tablet may be in a range of about 1 millimeters (mm) to about 6 mm, about 1 mm to about 5 mm, about 1 mm to about 4 mm, or about 1.0 mm to about 3 mm. When a diameter of the mini-tablet is less than 1 mm, it may be difficult to manufacture a punch for tablet preparation, the punch may be broken because the punch is thinned, and tablet discharge failure may occur in a tablet machine. When a diameter of the mini-tablet is greater than 10 mm, dissolution may be slow under a dissolution condition of a slow stirring rate, which may result in excessive dissociation of esomeprazole or proton pump inhibitors (PPI) by gastric acid having a low pH, consequently inducing a decrease in bioavailability of the drug. When a capsule is packed with a mini-tablet having a diameter of 10 mm or greater, the convenience of patient's medication may be deteriorated due to an increased size of the capsule, and as a content of main ingredients contained in one tablet is increased, a high- or low-content capsule may be generated due to a packing error. In addition, when a mini-tablet is coated on a double release formulation, a tablet having a diameter of 10 mm or greater may not smoothly flow in a fluidized bed coater, thus adversely affecting stability of the formulation due to difficulties in a coating process, unevenness of coating, and harsh coating conditions.

The term "core" as used herein may be used interchangeably with the term "nucleus". The core may include esomeprazole or a pharmaceutically acceptable salt thereof, which is a pharmaceutically active material.

The dissolution test is performed using a paddle test. The dissolution test is performed in the presence of a simulated intestinal fluid (medium). A pH of the simulated intestinal fluid may be in a range of about 6.5 to about 7.0 or about 6.7 to about 6.9. The dissolution test may be performed at about 20 revolutions per minute (rpm) to about 30 rpm. The dissolution test may be performed at a temperature in a range of about 30°C to about 40°C, about 32°C to about 40°C, about 34°C to about 40°C, or about 36°C to about 40°C, or at 37°C.

In the pharmaceutical composition, esomeprazole or a pharmaceutically acceptable salt thereof may be dissolved by about 50% or more within about 15 minutes, or about 10 minutes of a dissolution test. The pharmaceutical composition may be a rapid-release composition.

A disintegration time of the core may be within about 1 minute, about 45 seconds, about 30 seconds, about 25 seconds, about 20 seconds, or about 18 seconds. For example, a disintegration time of the core may be within about 30 seconds. In the pharmaceutical composition, the core may rapidly be disintegrated within a short time, thereby improving stability of the pharmaceutical composition.

The core may further include at least one excipient selected from a diluent, a disintegrant, a binder, a lubricant, a surfactant, an antioxidant, a preservative, and a stabilizer.

The diluent may be at least one selected from mannitol, microcrystalline cellulose, lactose, cellulose and a derivative, dibasic or tribasic basic calcium phosphate, erythritol, pregelatinized starch, sorbitol, and xylitol. For example, the diluent may be mannitol and microcrystalline cellulose. A content of the diluent may be in a range of about 1 percent by weight (wt%) to about 50 wt%, about 10 wt% to about 40 wt%, or about 20 wt% to about 40 wt%, based on a total content of the core.

The disintegrant is low-substituted hydroxypropyl cellulose being in a range of about 12 wt% to about 30 wt%, based on a total content of the core.

The binder may be at least one selected from the group consisting of hydroxypropyl cellulose (HPC), copovidone (a copolymer of vinylpyrrolidone and another vinyl derivative), hydroxypropyl methylcellulose (HPMC), polyvinyl pyrrolidone (povidone), pregelatinized starch, and low substituted hydroxypropyl cellulose. For example, the binder may be HPC. A content of the binder may be in a range of about 1 wt% to about 30 wt%, about 1 wt% to about 20 wt%, or about 1 wt% to about 10 wt%, based on a total content of the core.

The lubricant may be at least one selected from sodium stearyl fumarate, magnesium stearate, talc, polyethylene glycol, calcium behenate, calcium stearate, and hydrogenated castor oil. For example, the lubricant may be sodium stearyl fumarate. A content of the lubricant may be in a range of about 1 wt% to about 30 wt%, about 1 wt% to about 20 wt%, or about 1 wt% to about 10 wt%, based on a total content of the core.

The antioxidant may be at least one selected from butylated hydroxytoluene (BHT), butylated hydroxyanisole (BHA), ascorbic acid, ascorbyl palmitic acid, ethylenediamine tetraacetic acid (EDTA), and sodium pyrosulfate. A content of the antioxidant may be in a range of about 1 wt% to about 30 wt%, about 1 wt% to about 20 wt%, or about 1 wt% to about 10 wt%, based on a total content of the core.

The stabilizer may be an antioxidant, an acidifying agent, or a basicizing agent. A content of the stabilizer may be in a range of about 1 wt% to about 30 wt%, about 1 wt% to about 20 wt%, or about 1 wt% to about 10 wt%, based on a total content of the core.

The core may be coated with an enteric coating layer, a slow-release coating layer, or a combination thereof.

The core may be packing in a capsule. The capsule may be any suitable capsule commonly used in the art. The capsule may be a hard capsule or a soft capsule. The number of the cores packed in a capsule may vary depending on a needed dosage of a patient. A capsule may include about 5 cores to about 80 cores, about 5 cores to about 40 cores, or about 5 cores to about 20 cores. In the pharmaceutical composition including the core packed in a capsule, the core may be completely disintegrated about 1 minute, about 45 seconds, about 30 seconds, about 25 seconds, about 20 seconds, or about 18 seconds, after disintegration of the capsule. By coating with cores or core tablets each having different sizes, esomeprazole drug may have a double release pattern.

The pharmaceutical composition may be for prevention and treatment of gastroesophageal reflux disease or diseases related to excessive secretion of gastric acid. The pharmaceutical composition may be a pharmaceutical composition for prevention and treatment of reflux esophagitis, gastritis, duodenitis, gastric ulcer, duodenal ulcer, and peptic ulcer. The term "prevention" means all of the actions by which the occurrence of the disease is retarded by the administration of the pharmaceutical composition. The term "treatment" means all of the actions by which the symptoms of the disease have taken a turn for the better or been modified favorably by administration of the pharmaceutical composition.

The pharmaceutical composition may be a composition for oral administration. An administration dose of the pharmaceutical composition may be, for example, in a range of about 0.001 milligrams per kilogram (mg/kg) to about 100 mg/kg, about 0.01 mg/kg to about 10 mg/kg, or about 0.1 mg/kg to about 1 mg/kg for adults, once per day, several times per day, once per week, once per 2 weeks, once per 3 weeks, once per 4 weeks, or once per year.

Another aspect provides a method of preparing the pharmaceutical composition according to an aspect, the method including: preparing a mixture by mixing esomeprazole or a pharmaceutically acceptable salt thereof with at least one excipient selected from a diluent, a disintegrant, a binder, a lubricant, a surfactant, an antioxidant, a preservative, and a stabilizer; and

dry-granulating and tableting the mixture to obtain a core in a form of a multi-unit spheroidal tablet (MUST).

Esomeprazole, a pharmaceutically acceptable salt thereof, a diluent, a disintegrant, a binder, a lubricant, a surfactant, an antioxidant, a preservative, a stabilizer, a dry granule, a MUST, a core, and a pharmaceutical composition are defined the same as above.

The preparing of a mixture by mixing esomeprazole or a pharmaceutically acceptable salt thereof with at least one excipient selected from a diluent, a disintegrant, a binder, a lubricant, a surfactant, an antioxidant, a preservative, and a stabilizer may be performed simultaneously or sequentially.

The method may further include coating the core with an enteric coating layer, a slow-release coating layer, or a combination thereof. The coating may be performed using a method known in the art.

The method may further include packing a capsule with the core. The packing may be performed using a method known in the art.

Another aspect provides a method of preventing or treating gastroesophageal reflux disease or diseases related to excessive secretion of gastric acid including administering to a subject the pharmaceutical composition according to an aspect.

### Advantageous Effects of Invention

According to a pharmaceutical composition containing esomeprazole or a pharmaceutically acceptable salt thereof according to an aspect and a method of preparing the pharmaceutical composition, in case that the pharmaceutical composition is developed as the foregoing multi-unit spheroidal tablet (MUST), the dissolution may be accelerated in a condition of a slow stirring rate than that of a conventional pellet formulation or single tablet. Also, proton pump inhibitors (PPI)-based drugs including esomeprazole may be dissociated by gastric acid having a low pH, consequently inducing a decrease in bioavailability of the drug, but the pharmaceutical composition according to this may be minimize this.

### Brief Description of Drawings

These and/or other aspects will become apparent and more readily appreciated from the following description of the embodiments, taken in conjunction with the accompanying drawings in which:
FIG.1A is a graph of a dissolution rate (percent, %) of esomeprazole versus dissolution time (minutes, mins) of the formulations prepared in Examples 1 to 3 and Comparative Examples 1 to 6;
FIG.1B is a graph of a dissolution rate (%) of esomeprazole versus dissolution time (mins) of the formulations prepared in Examples 1, 4, and 5 and Comparative Example 8, varying in terms of multi-unit spheroidal tablet (MUST) punch diameter;
FIG. 1C is a graph of a dissolution rate (%) of esomeprazole versus dissolution time (mins) of the tablet formulation (Example 1) and the pellet formulation; and
FIG. 2 is a graph of disintegration time (seconds, sec) of the formulations prepared in Examples 1 to 3 and Comparative Examples 1 to 6.

### Mode for the Invention

Hereinafter, the present disclosure will be described in further detail with reference to Examples. However, these examples are not intended to limit the scope of the one or more embodiments of the present disclosure.

### Example 1: Preparation of tablet esomeprazole magnesium salt

As in Table 1, first, esomeprazole magnesium salt was mixed with mannitol, and then was sieved through a 30-mesh round sieve. The prepared mixture, low-substituted hydroxypropyl cellulose, croscarmellose sodium, hydroxypropyl cellulose, and sodium stearyl fumarate were added to an empty blender, followed by mixing for 15 minutes, thereby preparing a final mixture. The final mixture was added to a roller compactor for dry granulation. The obtained granules were sieved through a 30-mesh round sieve.

Subsequently, tablets having a hardness of about 1 kiloponds (kp) to about 2 kp and a weight of about 75 milligrams (mg) per 10 tablets were prepared by a tablet machine using a MUST punch having a diameter of 2.0 mm.

**[Table 1]**

| Ingredient | Weight (mg/10 tablets) | Percent by weight (%) |
|---|---|---|
| Esomeprazole magnesium salt trihydrate | 22.3 | 29.7 |
| Mannitol | 28.7 | 38.3 |
| Low-substituted hydroxypropyl cellulose | 13.8 | 18.4 |
| Croscarmellose sodium | 4.8 | 6.4 |
| Hydroxypropyl cellulose | 2.4 | 3.2 |
| Sodium stearyl fumarate | 3.0 | 4.0 |
| **Total** | **75.0** | **100.0** |

### Examples 2 and 3: Preparation of tablet esomeprazole magnesium salt

Tablets were prepared in substantially the same manner as in Example 1, except that the amount of low-substituted hydroxypropyl cellulose was different. The compositions of the prepared tablets are shown in Table 2.

**[Table 2]**

| Ingredient | Example 1(mg/10 tablets) | Example 2(mg/10 tablets) | Example 3(mg/10 tablets) |
|---|---|---|---|
| Esomeprazole magnesium salt trihydrate | 22.3 | 22.3 | 22.3 |
| Mannitol | 28.7 | 32.9 | 24.5 |
| Low-substituted hydroxypropyl cellulose | 13.8 | 9.6 | 18 |
| Croscarmellose sodium | 4.8 | 4.8 | 4.8 |
| Hydroxypropyl cellulose | 2.4 | 2.4 | 2.4 |
| Sodium stearyl fumarate | 3.0 | 3.0 | 3.0 |
| **Total** | **75.0** | **75.0** | **75.0** |

### Examples 4 and 5: Preparation of tablet esomeprazole magnesium salt

Tablets were prepared in substantially the same manner as in Example 1 with the same composition as Example 1, except that the diameter of the MUST punch was different.
- Example 4: Tablets were prepared using a MUST punch having a diameter of 1 mm such that a weight of 40 tablets was about 75 mg.
- Example 5: Tablets were prepared using a MUST punch having a diameter of 4 mm such that a weight of 2 tablets was about 75 mg.

### Comparative Example 1: Preparation of tablet esomeprazole magnesium salt

As in Table 3, first, esomeprazole magnesium salt was mixed with mannitol, and then was sieved through a 30-mesh round sieve. The prepared mixture, microcrystalline cellulose, croscarmellose sodium, hydroxypropyl cellulose, and sodium stearyl fumarate were added to an empty blender, followed by mixing for 15 minutes, thereby preparing a final mixture. The final mixture was added to a roller compactor for dry granulation. The obtained granules were sieved through a 20-mesh round sieve.

Subsequently, tablets having a hardness of about 1 kp to about 2 kp and a weight of about 75 mg per 10 tablets were prepared by a tablet machine using a MUST punch having a diameter of 2.0 mm.

**[Table 3]**

| Ingredient | Weight (mg/10 tablets) | Percent by weight (%) |
|---|---|---|
| Esomeprazole magnesium salt trihydrate | 22.3 | 29.7 |
| Mannitol | 29.7 | 39.6 |
| Microcrystalline cellulose | 15.2 | 20.3 |
| Croscarmellose sodium | 2.4 | 3.2 |
| Hydroxypropyl cellulose | 2.4 | 3.2 |
| Sodium stearyl fumarate | 3.0 | 4.0 |
| **Total** | **75.0** | **100.0** |

### Comparative Examples 2 to 6: Preparation of tablet esomeprazole magnesium salt

Tablets were prepared in substantially the same manner as in Example 1, except that the amount of low-substituted hydroxypropyl cellulose were different. The compositions thereof are shown in Table 4.

**[Table 4]**

| Ingredient | Comp. Example 2(mg/10 tablets) | Comp. Example 3(mg/10 tablets) | Comp. Example 4(mg/10 tablets) | Comp. Example 5(mg/10 tablets) | Comp. Example 6(mg/10 tablets) |
|---|---|---|---|---|---|
| Esomeprazole magnesium salt trihydrate | 22.3 | 22.3 | 22.3 | 22.3 | 22.3 |
| Mannitol | 27.3 | 24.3 | 37.7 | 19.5 | - |
| Lactose hydrate | - | - | - | - | 27.3 |
| Microcrystalline cellulose | 15.2 | - | - | - | 15.2 |
| Anhydrous dibasic potassium phosphate | - | 13.8 | - | - | - |
| Low-substituted hydroxypropyl cellulose | - | - | 4.8 | 23.0 | - |
| Croscarmellose sodium | 4.8 | 4.8 | 4.8 | 4.8 | 4.8 |
| Hydroxypropyl cellulose | 2.4 | 2.4 | 2.4 | 2.4 | 2.4 |
| Sodium stearyl fumarate | 3.0 | 3.0 | 3.0 | 3.0 | 3.0 |
| **Total** | **75.0** | **75.0** | **75.0** | **75.0** | **75.0** |

### Comp. Example: comparative Example)

### Comparative Examples 7 and 8: Preparation of tablet esomeprazole magnesium salt

Tablets were prepared in substantially the same manner as in Example 1 with the same composition as Example 1, except that the diameter of the MUST punch was different.
- Comparative Example 7: Tablets were prepared using a MUST punch having a diameter of 0.7 mm such that a weight of 75 tablets was about 75 mg.
- Comparative Example 8: Tablets were prepared using a MUST punch having a diameter of 7.0 mm such that a weight of 1 tablet was about 75 mg.

### Experimental Example 1. Dissolution test of esomeprazole preparation

### (1) Comparison of Examples 1 to 5 with Comparative Examples 1 to 8

Under the dissolution conditions and analysis conditions described herein, the dissolution rates of esomeprazole of the formulations of Examples 1 to 5 and Comparative Examples 1 to 8 were measured.

### <Dissolution conditions>

Eluent: 900 milliliters (mL) of simulated intestinal fluid (pH 6.8)
Apparatus: Paddle test, 25 rpm
Temperature: 37°C
Dissolution time: 5 minutes, 10 minutes, 15 minutes, 30 minutes, and 45 minutes (max)
(After a lapse of 30 minutes, the stirring rate was adjusted to 150 rpm)

### <Analysis conditions>

Used device: high-performance liquid chromatography (HPLC, Hitachi 5000 series, Japan)
Detector: Ultraviolet absorptiometer (measured wavelength: 302 nanometers (nm))
Column: a column of a stainless still pipe having an inner diameter of about 4.0 mm and a length of about 10 cm, which is packed with silica gel having a particle diameter of 5 *µ*m for liquid chromatography
Mobile phase: sodium phosphate buffer solution (pH 7.3): acetonitrile: distilled water = 50:35:15
Flow rate: 1.0 milliliter per minute (mL/min)
Column temperature: 30°C

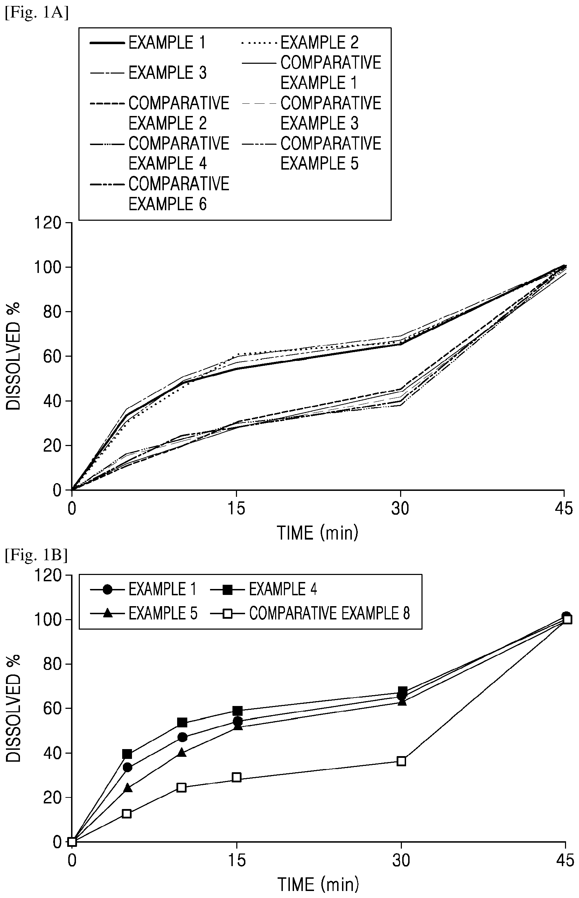

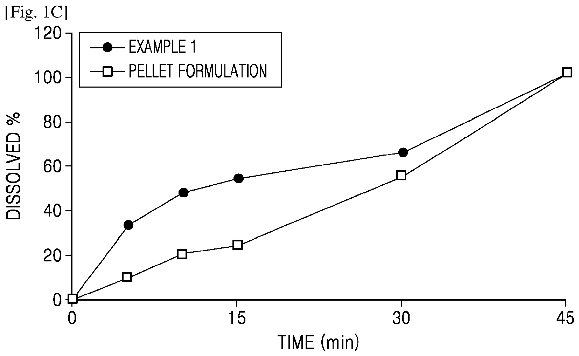

The measured dissolution rate of esomeprazole are shown in Table 5, Table 6, and FIG. 1A.

**[Table 5]**

| | **Dissolution rate of esomeprazole (%)** | | | | | | | | | |
|---|---|---|---|---|---|---|---|---|---|---|
| | Example 1 | | Example 2 | | Example 3 | | Example 4 | | Example 5 | |
| Time (mins) | AVG. | S.D. | AVG. | S.D. | AVG. | S.D. | AVG. | S.D. | AVG. | S.D. |
| 0 | 0.0 | 0.0 | 0.0 | 0.0 | 0.0 | 0.0 | 0 | 0 | 0 | 0 |
| 5 | 33.6 | 2.4 | 30.7 | 2.7 | 36.7 | 2.9 | 39.8 | 1.4 | 24.3 | 4.2 |
| 10 | 47.7 | 3.7 | 45.8 | 1.6 | 50.8 | 3.1 | 53.3 | 1.7 | 40.3 | 2.8 |
| 15 | 54.0 | 2.1 | 59.8 | 1.8 | 60.3 | 1.3 | 58.8 | 2.2 | 52.1 | 2.3 |
| 30 | 66.1 | 1.3 | 67.1 | 2.1 | 68.9 | 1.2 | 67.2 | 0.6 | 63.2 | 2.6 |
| 45(M ax) | 101.4 | 1.0 | 99.6 | 2.0 | 98.9 | 1.4 | 99.4 | 0.4 | 99.5 | 0.2 |

### (AVG.: average, S.D.: standard deviation)

**[Table 6]**

| | **Dissolution rate of esomeprazole (%)** | | | | | | | | | | | | | |
|---|---|---|---|---|---|---|---|---|---|---|---|---|---|---|
| | Comp.Exa mple 1 | | Comp.Exa mple 2 | | Comp. Example 3 | | Comp. Example 4 | | Comp. Example 5 | | Comp. Example 6 | | Comp.Exa mple 8 | |
| Time (mins ) | AV G. | S.D. | AV G. | S.D. | AV G. | S.D. | AV G. | S.D. | AV G. | S.D. | AV G. | S.D. | AV G. | S.D. |
| 0 | 0.0 | 0.0 | 0.0 | 0.0 | 0.0 | 0.0 | 0.0 | 0.0 | 0 | 0 | 0.0 | 0.0 | 0 | 0 |
| 5 | 11.6 | 6.5 | 10.9 | 3.8 | 15.6 | 1.3 | 14.3 | 1.9 | 31.2 | 2.1 | 13.1 | 1.9 | 13.2 | 7.6 |
| 10 | 20.0 | 4.9 | 19.9 | 4.6 | 22.4 | 2.8 | 22.8 | 2.7 | 48.3 | 2.6 | 24.8 | 3.1 | 24.7 | 5.6 |
| 15 | 27.7 | 6.4 | 30.6 | 3.6 | 28.7 | 2.9 | 28.9 | 3.7 | 57.7 | 2.3 | 27.8 | 2.7 | 28.6 | 4.5 |
| 30 | 43.9 | 3.8 | 45.9 | 3.4 | 41.8 | 3.0 | 37.9 | 1.9 | 68.2 | 2.7 | 40.2 | 2.5 | 36.6 | 4.5 |
| 45 (Max ) | 97.5 | 2.9 | 100. 6 | 2.4 | 98.9 | 1.8 | 100. 8 | 2.2 | 99.5 | 1.1 | 100. 1 | 1.4 | 99.4 | 0.4 |

### (Comp. Example: comparative Example, AVG.: average, S.D.: standard deviation)

As shown in Tables 5 and 6 and FIGS. 1A and 1B, the formulations of Examples 1 to 5 and Comparative Examples 5 exhibited rapid release characteristics of drugs, whereas the formulations of Comparative Examples 1 to 4, 6, and 8 exhibited low dissolution rates as well as slow disintegration of the tablets.

In the case of the formulation of Comparative Example 7, insufficient packing of granules in a hole die was observed with naked eye in a tableting process, and difficulties were found in the tableting process because an edge of the punch was broken by a slight error in assembling of equipment.

When a dissolution rate of the drug is lowered due to the delayed disintegration time, the drug may be dissociated by gastric acid before absorbed into a living matter, thus decreasing bioavailability of the drug. To overcome this end, rapid disintegration of a tablet is required. In the case of the formulations of Examples 1 to 3, the drugs were rapidly dissolved, and thus the drugs were absorbed into a living matter, without dissociation by gastric acid or excretion of the drug. In contrast, in the case of the formulations of Comparative Examples 1 to 6, it was found that the formulations of Comparative Examples 1 to 6 were not suitable because disintegration of the drugs was delayed, which may result in excretion of the drug from a living matter.

### (2) Comparison of tablet formulation with pellet formulation

To compare dissolution rates of the formulations of esomeprazole, the tablet formulation of Example 1 and a pellet formulation of esomeprazole (esomeprazole 20 mg) were prepared. As for the pellet formulation of esomeprazole, 20 mg of Nexium (available from Astrazeneca), which is currently commercially available in the market, was purchased for the comparison test.

The dissolution rates of esomeprazole were measured following the method described in Experimental Example 1.(1). The results thereof are shown in Table 7 and FIG. 1B.

**[Table 7]**

| | **Example 1** | | **Pellet formulation (esomeprazole)** | |
|---|---|---|---|---|
| Time (mins) | Average | Standard deviation | Average | Standard deviation |
| 0 | 0.0 | 0.0 | 0.0 | 0.0 |
| 5 | 33.6 | 2.4 | 9.8 | 5.3 |
| 10 | 47.7 | 3.7 | 19.9 | 2.9 |
| 15 | 54.0 | 2.1 | 24.0 | 3.1 |
| 30 | 66.1 | 1.3 | 54.9 | 2.9 |
| 45 (Max) | 101.4 | 1.0 | 101.3 | 1.3 |

As shown in Table 7 and FIG. 1B, the tablet formulation of Example 1 was found to have rapid release characteristics, as compared with the pellet formulation. In particular, after a lapse of about 15 minutes, the dissolution rate of the pellet formulation was about 24 %, however, the dissolution rate of the tablet formulation was about 54 %, which is about 2 or more times higher than that of the pellet formulation.

### Experimental Example 2. Disintegration test of esomeprazole preparation

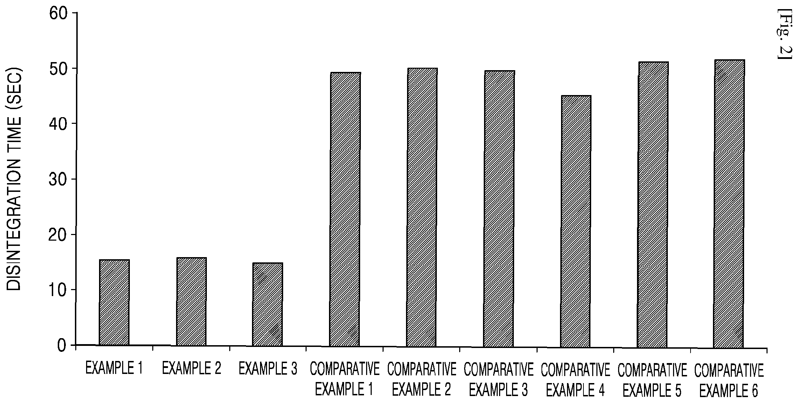

To compare disintegration times of the formulations of Examples 1 to 3 with the formulations of Comparative Examples 1 to 6, an experiment was performed according to the disintegration test method for general release preparations described in Korean Pharmacopoeia (KP). The disintegration time of each of the formulations was measured, and the results thereof are shown in Table 8 and FIG. 2.

**[Table 8]**

| **Formulation** | **Disintegration time (sec)** |
|---|---|
| Example 1 | 15.3 |
| Example 2 | 16.1 |
| Example 3 | 14.9 |
| Example 4 | 10.4 |
| Example 5 | 25.6 |
| Comparative Example 1 | 49.6 |
| Comparative Example 2 | 50.3 |
| Comparative Example 3 | 49.9 |
| Comparative Example 4 | 45.7 |
| Comparative Example 5 | 13.1 |
| Comparative Example 6 | 52.1 |
| Comparative Example 8 | 63.1 |

As shown in Table 8 and FIG. 2, the formulations of Example 1 to 3 and Comparative Example 1 to 6 are not commonly developed tablet formulations, the disintegration time of each of the formulations was within 1 minute, except the single tablet of Comparative Example 8. In particular, the formulations of Examples 1 to 5 each had a disintegration time within about 15 seconds.

To secure rapid release characteristics in a living matter, disintegration of the drug is required to be completed within about 30 seconds. Thus, the formulations of Examples 1 to 5 were found to be suitable in this regard.

### Experimental Example 3. Evaluation of physical properties of granules

The physical properties of the granules prepared in Examples 1 to 3 and Comparative Examples 1 to 6 were evaluated. When tableting, as 19 tips are tableted at once, flowability of granules is an important variable in securing mass deviation and uniformity of the tablets. The results of evaluation of physical properties are shown in Table 9.

**[Table 9]**

| **Formulation** | **Flowability of granules** (angle of repose °) |
|---|---|
| Example 1 | 38.0 |
| Example 2 | 39.0 |
| Example 3 | 39.5 |
| Comparative Example 1 | 36.0 |
| Comparative Example 2 | 35.1 |
| Comparative Example 3 | 37.9 |
| Comparative Example 4 | 37.9 |
| Comparative Example 5 | 42.3 |
| Comparative Example 6 | 38.8 |

As shown in Table 9, a suitable angle of repose for tableting was within 40°. As tableting of mini-tablets are required, a diameter of the punch may be small, and a diameter of a hole die, through which granules are packed, may also be small. As a small punch is used, the flowability of granules was found to be an important factor in producing tablets having a uniform mass and hardness. When an angle of repose is 40° or greater, uniform packing of granules is difficult, and thus, it is difficult to secure uniform mass and hardness of tablets. The formulations of Examples 1 to 3 were found to have satisfactory flowability of granules within 40°. On the other hand, the formulation of Comparative Example 5 had an angle of repose of 40° or greater, thus being improper in tableting uniform mini-tablets.

## Claims

1. A pharmaceutical composition comprising a core in a form of multi-unit spheroidal tablet (MUST) containing esomeprazole or a pharmaceutically acceptable salt thereof and a disintegrant,
wherein the disintegrant is low-substituted hydroxypropyl cellulose, the content of the low-substituted hydroxypropyl cellulose being in a range of about 12 percent by weight (wt%) to about 30 wt%, based on a total weight of the core,
wherein the pharmaceutical composition is dissolved by 50 percent (%) or more within 15 minutes of a dissolution test in presence of simulated intestinal fluid at 25 revolutions per minute (rpm), when a dissolution test takes place using the paddle method.

2. The pharmaceutical composition of claim 1, wherein the esomeprazole or a pharmaceutically acceptable salt thereof is in a form of a dry granule.

3. The pharmaceutical composition of claim 1, wherein a disintegration time of the core is within 30 seconds.

4. The pharmaceutical composition of claim 1, wherein the core further comprises at least one excipient selected from a diluent, a binder, a lubricant, a surfactant, an antioxidant, a preservative, and a stabilizer.

5. The pharmaceutical composition of claim 1, wherein a diameter or a longest diagonal length of the core is in a range of about 1.0 millimeter (mm) to about 6.0 mm.

6. The pharmaceutical composition of claim 1, wherein a diameter or a longest diagonal length of the core is in a range of about 1.0 mm to about 5.0 mm.

7. The pharmaceutical composition of claim 1, wherein a diameter or a longest diagonal length of the core is in a range of about 1.0 mm to about 3.0 mm.

8. A method of preparing the pharmaceutical composition of claim 1, the method comprising:
preparing a mixture by mixing esomeprazole or a pharmaceutically acceptable salt thereof with a disintegrant and optionally at least one excipient selected from a diluent, a binder, a lubricant, a surfactant, an antioxidant, a preservative, and a stabilizer; and
dry-granulating and tableting the mixture to obtain a core in a form of a multi-unit spheroidal tablet (MUST),
wherein the disintegrant is low-substituted hydroxypropyl cellulose, the content of the low-substituted hydroxypropyl cellulose being in a range of about 12 percent by weight (wt%) to about 30 wt%, based on a total weight of the core.

## Patentansprüche

1. Pharmazeutische Zusammensetzung, umfassend einen Kern in Form einer kugelförmigen Tablette mit mehreren Einheiten (MUST), die Esomeprazol oder ein pharmazeutisch akzeptables Salz davon und ein Zerfallsmittel enthält,
wobei das Zerfallsmittel niedrig substituierte Hydroxypropylcellulose ist, wobei der Gehalt der niedrig substituierten Hydroxypropylcellulose in einem Bereich von ungefähr 12 Gewichtsprozent (Gew.-%) bis ungefähr 30 Gew.-%, bezogen auf ein Gesamtgewicht des Kerns, liegt,
wobei die pharmazeutische Zusammensetzung um 50 Prozent (%) oder mehr innerhalb von 15 Minuten eines Auflösungstests in Gegenwart von simuliertem Darmfluid bei 25 Umdrehungen pro Minute (U/min) aufgelöst wird, wenn ein Test zur Bestimmung der Auflösungsgeschwindigkeit unter Verwendung der Blattrührer-Methode stattfindet.

2. Pharmazeutische Zusammensetzung nach Anspruch 1, wobei das Esomeprazol oder ein pharmazeutisch akzeptables Salz davon in Form eines trockenen Granulats vorliegt.

3. Pharmazeutische Zusammensetzung nach Anspruch 1, wobei eine Zerfallszeit des Kerns innerhalb von 30 Sekunden liegt.

4. Pharmazeutische Zusammensetzung nach Anspruch 1, wobei der Kern ferner mindestens einen Hilfsstoff umfasst, der aus einem Verdünnungsmittel, einem Bindemittel, einem Schmiermittel, einem oberflächenaktiven Mittel, einem Antioxidationsmittel, einem Konservierungsmittel und einem Stabilisator ausgewählt ist.

5. Pharmazeutische Zusammensetzung nach Anspruch 1, wobei ein Durchmesser oder eine längste diagonale Länge des Kerns in einem Bereich von ungefähr 1,0 Millimeter (mm) bis ungefähr 6,0 mm liegt.

6. Pharmazeutische Zusammensetzung nach Anspruch 1, wobei ein Durchmesser oder eine längste diagonale Länge des Kerns in einem Bereich von ungefähr 1,0 mm bis ungefähr 5,0 mm liegt.

7. Pharmazeutische Zusammensetzung nach Anspruch 1, wobei ein Durchmesser oder eine längste diagonale Länge des Kerns in einem Bereich von ungefähr 1,0 mm bis ungefähr 3,0 mm liegt.

8. Verfahren zur Vorbereitung der pharmazeutischen Zusammensetzung nach Anspruch 1, wobei das Verfahren Folgendes umfasst:
Vorbereiten eines Gemischs durch Mischen von Esomeprazol oder eines pharmazeutisch akzeptablen Salzes davon mit einem Zerfallsmittel und optional mindestens einem Hilfsstoff, ausgewählt aus einem Verdünnungsmittel, einem Bindemittel, einem Schmiermittel, einem oberflächenaktiven Mittel, einem Antioxidationsmittel, einem Konservierungsmittel und einem Stabilisator; und
Trockengranulieren und Tablettieren des Gemischs, um einen Kern in Form einer kugelförmigen Tablette mit mehreren Einheiten (MUST) zu erzielen,
wobei das Zerfallsmittel niedrig substituierte Hydroxypropylcellulose ist, wobei der Gehalt der niedrig substituierten Hydroxypropylcellulose in einem Bereich von ungefähr 12 Gewichtsprozent (Gew.-%) bis ungefähr 30 Gew.-%, bezogen auf ein Gesamtgewicht des Kerns, liegt.

## Revendications

1. Composition pharmaceutique comprenant un noyau sous forme d'un comprimé sphéroïdal à unités multiples (MUST) contenant de l'ésoméprazole ou un sel pharmaceutiquement acceptable de celui-ci et un désintégrant,
dans lequel le désintégrant est une hydroxypropylcellulose faiblement substituée, la teneur en hydroxypropylcellulose faiblement substituée étant dans une plage comprise entre environ 12 pour cent en poids (% en poids) et environ 30 % en poids, sur la base d'un poids total du noyau,
dans lequel la composition pharmaceutique est dissoute de 50 pour cent (%) ou plus dans les 15 minutes d'un essai de dissolution en présence d'un fluide intestinal simulé à 25 tours par minute (trs/mn), lorsqu'un essai de dissolution a lieu à l'aide d'une méthode utilisant un appareil à palette.

2. Composition pharmaceutique selon la revendication 1, dans laquelle l'ésoméprazole ou un sel pharmaceutiquement acceptable de celui-ci est sous une forme de granule sec.

3. Composition pharmaceutique selon la revendication 1, dans laquelle un temps de désintégration du noyau est compris dans les 30 secondes.

4. Composition pharmaceutique selon la revendication 1, dans laquelle le noyau comprend en outre au moins un excipient sélectionné parmi un diluant, un liant, un lubrifiant, un tensioactif, un anti-oxydant, un conservateur, et un stabilisateur.

5. Composition pharmaceutique selon la revendication 1, dans laquelle un diamètre ou une longueur en diagonale la plus longue du noyau est dans la plage d'environ 1,0 millimètre (mm) à environ 6,0 mm.

6. Composition pharmaceutique selon la revendication 1, dans laquelle un diamètre ou une longueur en diagonale la plus longue du noyau est dans la plage d'environ 1,0 mm à environ 5,0 mm.

7. Composition pharmaceutique selon la revendication 1, dans laquelle un diamètre ou une longueur en diagonale la plus longue du noyau est dans une plage d'environ 1,0 mm à environ 3,0 mm.

8. Procédé de préparation de la composition pharmaceutique selon la revendication 1, le procédé comprenant :
la préparation d'un mélange en mélangeant de l'ésoméprazole ou un sel pharmaceutiquement acceptable de celui-ci avec un désintégrant et facultativement au moins un excipient sélectionné parmi un diluant, un liant, un lubrifiant, un tensioactif, un anti-oxydant, un conservateur, et un stabilisateur; et
la granulation à sec et la mise en comprimés du mélange pour obtenir un noyau sous forme d'un comprimé sphéroïdal à unités multiples (MUST),
dans lequel le désintégrant est une hydroxypropylcellulose faiblement substituée, la teneur de l'hydroxypropylcellulose faiblement substituée étant dans une plage d'environ 12 pour cent en poids (% en poids) à environ 30 % en poids, sur la base d'un poids total du noyau.
